# FASCICULE DE BREVET EUROPEEN

(11) **EP 1 985 363 B1**
(45) Date de publication et mention de la délivrance du brevet: **01.09.2010**
(21) Numéro de dépôt: 08290313.9
(22) Date de dépôt: 31.03.2008
(51) Int. Cl.: B01J 29/74, C07C 5/27, B01J 37/10

(54) **Zéolithe EU-1 modifiée et son utilisation en isomérisation des composés C8 aromatiques**
Modifizierter EU-1-Zeolith und seine Anwendung bei der Isomerisation von aromatischen C8-Verbindungen
Modified EU-1 zeolite and its use for isomerising aromatic C8 compounds

(30) Priorité: 23.04.2007 FR 0702943
(43) Date de publication de la demande: 29.10.2008
(73) Titulaire: IFP, 92852 Rueil-Malmaison Cédex (FR)
(72) Inventeur: Guillon, Emmanuelle, 69390 Vernaison (FR); Sanchez, Eric, 69230 Saint Genis Laval (FR)

(56) Documents cités:
- FR-A- 2 765 209
- US-A- 4 695 667
- RAO G N ET AL: "THERMAL AND HYDROTHERMAL STABILITIES OF ZEOLITE EU-1" APPLIED CATALYSIS A: GENERAL, ELSEVIER SCIENCE, AMSTERDAM, NL, vol. 119, 1994, pages 33-43, XP002055790 ISSN: 0926-860X
- BREUNINGER M.: "In-situ-infrarotspektroskopische Untersuchungen zur Cumolsynthese an sauren Zeolithkatalysatoren" OPUS ELEKTRONISCHE DISSERTATIONEN, [Online] 3 mars 2004 (2004-03-03), XP002459438 Institut für Technische Chemie der Universität Stuttgart Extrait de l'Internet: URL:http://elib.uni-stuttgart.de/opus/voll texte/2004/2095/pdf/DISS_MBreuninger.pdf> [extrait le 2007-11-29]

## Description

La présente invention se rapporte à un catalyseur comprenant une zéolithe EU-1 dont la charpente zéolithique a été modifiée. Plus précisément ladite zéolithe EU-1 présente des atomes d'aluminium tétracoordinés et des atomes d'aluminium hexacoordinés dans une proportion pondérale bien déterminée. Ledit catalyseur trouve avantageusement son application dans un procédé d'isomérisation d'une coupe aromatique comprenant au moins un composé aromatique à huit atomes de carbone par molécule. Ladite coupe est une charge contenant un mélange de xylènes, de l'éthylbenzène ou un mélange de xylènes et d'éthylbenzène. Cette charge est communément appelée "coupe C8 aromatiques".

### Etat de la technique antérieure

La catalyse de l'isomérisation d'une coupe C8 aromatiques, par exemple de l'éthylbenzène, en xylènes a fait l'objet de nombreux brevets. Plusieurs catalyseurs zéolithiques ont été proposés pour catalyser une telle réaction. Parmi les zéolithes utilisées en isomérisation d'une coupe C8 aromatiques, on trouve la ZSM-5, utilisée seule ou en mélange avec d'autres zéolithes, comme par exemple la mordénite. Ces catalyseurs sont notamment décrits dans les brevets US-A-4 467 129, US-A-4 482 773 et EP-B-13 617. D'autres catalyseurs principalement à base de mordénite ont été décrits par exemple dans les brevets US-A-4 723 051, US-A-4 665 258 et FR-A-2 477 903. Plus récemment, il a été proposé un catalyseur à base d'une zéolithe de type structural EUO (EP-A1-923 987) ainsi qu'un catalyseur à base d'une zéolithe de type structural MTW (WO -A-2005/065.380).
La plupart de ces catalyseurs zéolithiques, bien qu'ils conduisent à une sélectivité en xylènes intéressante, ne permettent pas d'observer un rendement en xylènes optimisé en raison d'une conversion de la coupe C8 aromatique, notamment de l'éthylbenzène, trop faible.
Aussi, un des objectifs de la présente invention est de fournir un nouveau catalyseur à base d'une zéolithe EU-1, plus actif que les catalyseurs zéolithiques connus de l'art antérieur tout en étant aussi sélectif envers la production de xylènes, de manière à améliorer non seulement la conversion de la charge à isomériser mais également le rendement en produits désirés que constituent les xylènes.

### Résumé et intérêt de l'invention

La présente invention concerne un catalyseur comprenant au moins une zéolithe EU-1 modifiée contenant des atomes de silicium et des atomes d'aluminium, au moins une matrice et au moins un métal du groupe VIII de la classification périodique des éléments, ledit catalyseur étant caractérisé en ce que le nombre d'atomes d'aluminium hexacoordinés présents dans ladite zéolithe modifiée représente plus de 20% poids du nombre total d'atomes d'aluminium présents dans ladite zéolithe EU-1 modifiée.

Ledit catalyseur selon l'invention est avantageusement utilisé dans un procédé d'isomérisation d'une coupe aromatique comprenant au moins un composé aromatique à huit atomes de carbone par molécule.

Il a été découvert, de façon surprenante, qu'un catalyseur, sous forme de billes ou d'extrudés, comprenant au moins une matrice, au moins un métal du groupe VIII de la classification périodique des éléments, et au moins une zéolithe EU-1 dont le nombre d'atomes d'aluminium hexacoordinés représente plus de 20% poids du nombre total d'atomes d'aluminium présents dans ladite zéolithe modifiée, conduit à des performances catalytiques améliorées en terme de conversion d'une charge aromatique à isomériser lorsque ledit catalyseur est utilisé dans un procédé d'isomérisation d'une coupe aromatique comprenant au moins un composé aromatique à huit atomes de carbone par molécule. Un tel catalyseur est sensiblement plus actif qu'un catalyseur comprenant une zéolithe EU-1 non modifiée laquelle présente un nombre d'atomes d'aluminium hexacoordinés représentant moins de 20% poids du nombre total d'atomes d'aluminium présents dans la zéolithe non modifiée. Par ailleurs, la sélectivité en produits désirés, à savoir les xylènes, étant maintenue avec l'emploi d'un catalyseur comprenant une zéolithe EU-1 modifiée, il en résulte une augmentation du rendement en xylènes lorsque le procédé d'isomérisation est mis en oeuvre en présence du catalyseur selon l'invention.

### Description détaillée de l'invention

La présente invention porte sur un catalyseur comprenant au moins une zéolithe EU-1 modifiée contenant des atomes de silicium et des atomes d'aluminium, au moins une matrice et au moins un métal du groupe VIII de la classification périodique des éléments, ledit catalyseur étant caractérisé en ce que le nombre d'atomes d'aluminium hexacoordinés présents dans ladite zéolithe modifiée représente plus de 20% poids du nombre total d'atomes d'aluminium présents dans ladite zéolithe EU-1 modifiée.

Conformément à l'invention, la zéolithe EU-1 modifiée comprise dans le catalyseur selon l'invention présente un nombre d'atomes d'aluminium hexacoordinés, notés Alᵥₗ, représentant plus de 20% poids, préférentiellement plus de 22% poids, de manière plus préférée plus de 25% poids et de manière encore plus préférée plus de 30% poids, du nombre total d'atomes d'aluminium présents dans ladite zéolithe modifiée. Les atomes d'aluminium présents dans ladite zéolithe EU-1 comprise dans le catalyseur selon l'invention et ne se trouvant pas sous la forme d'aluminium hexacoordinés sont présents sous la forme d'atomes d'aluminium tétracoordinés notés Al_{IV}. Lesdits atomes d'aluminium tétracoordinés sont présents dans la charpente zéolithique alors que les atomes d'aluminium hexacoordinés sont présents hors de la charpente zéolithique (aluminium extra-réseau). En particulier, lesdits atomes d'aluminium hexacoordinés se trouvent, au moins en partie, dans la porosité de la zéolithe EU-1 modifiée.
Conformément à l'invention, le pourcentage pondéral des atomes d'aluminium tétracoordinés et hexacoordinés présents dans la zéolithe EU-1 modifiée est déterminé par résonance magnétique nucléaire du solide de ²⁷Al. La RMN de l'aluminium est en effet connue pour être utilisée en vue de repérer et de quantifier les différents états de coordination de ce noyau ("Analyse physico-chimiques des catalyseurs industriels", J. Lynch, Editions Technip (2001) chap. 13, pages 290 et 291). Le spectre RMN de l'aluminium de la zéolithe EU-1 modifiée présente deux signaux, l'un étant caractéristique de la résonance des atomes d'aluminium tétracoordinés et l'autre étant caractéristique de la résonance des atomes d'aluminium hexacoordinés. Les atomes d'aluminium tétracoordinés Al_{IV} résonnent à un déplacement chimique compris entre +40 ppm et +75 ppm et les atomes d'aluminium hexacoordinés Al_{VI} résonnent à un déplacement chimique compris entre -15 ppm et +15 ppm. Le pourcentage pondéral des deux espèces aluminiques Al_{IV} et Al_{VI} est quantifié par intégration des signaux correspondant à chacune de ces espèces.
Plus précisément, la zéolithe EU-1 modifiée présente dans le catalyseur selon l'invention a été analysée par RMN-MAS du solide ²⁷Al sur un spectromètre Brücker de type Avance 400 MHz à l'aide d'une sonde 4 mm optimisée pour l' ²⁷Al. La vitesse de rotation de l'échantillon est voisine de 14 kHz. L'atome d'aluminium est un noyau quadripolaire dont le spin est égal à 5/2. Dans des conditions d'analyse dites sélectives, à savoir un champs de radiofréquence faible égal à 30 kHz, un angle d'impulsion faible égal à π/2 et en présence d'un échantillon saturé en eau, la technique de RMN de rotation à l'angle magique (MAS), notée RMN-MAS, est une technique quantitative. La décomposition de chaque spectre RMN-MAS permet d'accéder directement à la quantité des différentes espèces aluminiques, à savoir des atomes d'aluminium tétracoordinés AI_{IV} et des atomes d'aluminium hexacoordinés AI_{VI}. Chaque spectre est calé en déplacement chimique par rapport à une solution 1 M de nitrate d'aluminium pour laquelle le signal d'aluminium est à zéro ppm. Les signaux caractérisant les atomes d'aluminium tétracoordinés Al_{IV} sont intégrés entre +40 ppm et +75 ppm ce qui correspond à l'aire 1 et les signaux caractérisant les atomes d'aluminium hexacoordinés AI_{VI} sont intégrés entre -15 ppm et +15 ppm ce qui correspond à l'aire 2. Le pourcentage pondéral des atomes d'aluminium hexacoordinés Al_{Vl} est égal au rapport aire 2/(aire 1 + aire 2).

La zéolithe EU-1 modifiée, comprise dans le catalyseur selon l'invention et contenant des atomes d'aluminium et des atomes de silicium, présente un rapport atomique Si/Al global compris entre 5 et 100, de préférence compris entre 10 et 50 et de manière très préférée compris entre 10 et 35. Conformément à l'invention, ledit rapport atomique Si/AI global de ladite zéolithe EU-1 modifiée ne varie avantageusement pas davantage de plus ou moins 2% du rapport atomique Si/Al global de la zéolithe EU-1 brute de synthèse dont est issue ladite zéolithe modifiée et de manière très avantageuse ledit rapport atomique Si/Al global de ladite zéolithe EU-1 modifiée reste inchangé par rapport à celui de la zéolithe EU-1 brute de synthèse. Le rapport atomique Si/Al global, déterminé par fluorescence X ou par absorption atomique, prend en comte aussi bien les atomes d'aluminium présents dans la charpente zéolithique que les atomes d'aluminium éventuellement présents hors de la charpente zéolithique encore appelé aluminium extra-réseau. Chaque atome d'aluminium tétracoordiné présent dans la charpente zéolithique de la zéolithe EU-1 modifiée est lié à quatre atomes d'oxygène et se trouve dans une configuration tétraédrique. Chaque atome d'aluminium hexacoordiné présent dans la zéolithe EU-1 modifiée, mais en dehors de la charpente zéolithique, est entouré de 6 atomes d'oxygène et se trouve dans une configuration octaédrique. Dans la zéolithe EU-1 brute de synthèse dont est issue la zéolithe EU-1 modifiée, le pourcentage pondéral des atomes d'aluminium tétracoordinés, déterminé par analyse RMN-MAS de l'aluminium comme décrit ci-dessus, est supérieur à 90%, de préférence supérieur à 95%, de manière très préférée supérieur à 98% et de manière encore plus préférée il est égal à 100%. Conformément à l'invention et selon un mode très préférée de réalisation de l'invention, le nombre global d'atomes d'aluminium présents dans la zéolithe EU-1 modifiée et celui des atomes d'aluminium présents dans la zéolithe EU-1 brute de synthèse dont est issue la zéolithe EU-1 modifiée sont identiques.

La zéolithe EU-1 modifiée, présente dans le catalyseur selon l'invention, se présente très avantageusement sous sa forme protonée (forme hydrogène H⁺) dans laquelle la proportion en cation autre que H⁺ est inférieure à 30% du nombre total de cations, de préférence inférieure à 20% et de manière très préférée inférieure à 5% par rapport au nombre total de cations sur la zéolithe.

Conformément à l'invention, ledit catalyseur comprend au moins un métal du groupe VIII choisi parmi le fer, le cobalt, le nickel, le ruthénium, le rhodium, le palladium, l'osmium, l'iridium et le platine, de préférence choisi parmi les métaux nobles du groupe VIII, de manière très préférée choisi parmi le palladium et le platine et de manière encore plus préférée il s'agit du platine. La dispersion du(es) métal(ux) du groupe VIII, déterminée par chimisorption, par exemple par titration H₂-O₂ ou par chimisorption du monoxyde de carbone, est comprise entre 50% et 100%, de préférence entre 60 % et 100% et de manière encore plus préférée entre 70% et 100%. Le coefficient de répartition macroscopique du(es) métal(ux) du groupe VIII, obtenu à partir de son profil déterminé par microsonde de Castaing, défini comme le rapport des concentrations du(es) métal(ux) du groupe VIII au coeur du grain par rapport au bord de ce même grain, est compris entre 0,7 et 1,3, de préférence entre 0,8 et 1,2. La valeur de ce rapport, voisine de 1, témoigne de l'homogénéité de la répartition du(es) métal(ux) du groupe VIII dans le catalyseur.

Ledit catalyseur comprend avantageusement au moins un métal additionnel choisi dans le groupe formé par les métaux des groupes IIIA, IVA et VIIB de la classification périodique des éléments et de préférence choisi parmi le gallium, l'indium, l'étain et le rhénium. Ledit métal additionnel est de préférence choisi parmi l'indium, l'étain et le rhénium.

Ledit catalyseur comprend également avantageusement du soufre.

Ledit catalyseur selon l'invention contient plus particulièrement :
- de 1 à 90%, de préférence de 3 à 80% et de manière encore plus préférée de 4 à 60% poids de ladite zéolithe EU-1 modifiée,
- de 0,01 à 4%, de préférence de 0,05 à 2% poids d'au moins un métal du groupe VIII de la classification périodique des éléments,
- éventuellement de 0,01 à 2%, de préférence de 0,05 à 1% poids d'au moins un métal additionnel choisi dans le groupe formé par les métaux des groupes IIIA, IVA et VIIB,
- éventuellement une teneur en soufre telle que le rapport du nombre d'atomes de soufre sur le nombre d'atome de(s) métal(ux) du groupe VIII soit compris entre 0,5 :1 et 2:1,
- au moins une matrice assurant le complément à 100% dans le catalyseur.

La matrice entrant dans la composition du catalyseur selon l'invention est choisie parmi les argiles, la magnésie, les alumines, les silices, l'oxyde de titane, l'oxyde de bore, la zircone, les phosphates d'aluminium, les phosphates de titane, les phosphates de zirconium, les silices-alumines et le charbon ou un mélange d'au moins deux de ces compositions. De préférence, la matrice est une alumine.

Ledit catalyseur se présente sous forme de billes ou d'extrudés, de préférence sous forme d'extrudés.

La présente invention a également pour objet la préparation du catalyseur selon l'invention. Une étape essentielle dans la mise en oeuvre de la préparation du catalyseur selon l'invention consiste à procéder à un traitement thermique, en présence de vapeur d'eau, sur une zéolithe EU-1 mise en forme ou non, brute de synthèse ou dépourvue du structurant organique. Ledit traitement thermique en présence de vapeur d'eau conduit à une modification de la charpente zéolithique de la zéolithe EU-1 soumise audit traitement de telle sorte que la zéolithe EU-1 modifiée présente un nombre d'atomes d'aluminium hexacoordinés représentant plus de 20% poids, de préférence plus de 22% poids, de manière plus préférée plus de 25% poids et de manière encore plus préférée plus de 30% poids, du nombre total d'atomes d'aluminium présents dans ladite zéolithe modifiée. De manière très préférée, la préparation du catalyseur selon l'invention ne comprend aucune étape dans laquelle la zéolithe EU-1 serait soumise à un traitement avec une solution acide.

Un premier mode de réalisation de la préparation du catalyseur selon l'invention consiste en un procédé comprenant au moins les étapes suivantes :
a1) la synthèse d'au moins une zéolithe EU-1 ayant un rapport atomique Si/AI global compris entre 5 et 100, de préférence entre 10 et 50 et de manière encore plus préférée entre 10 et 35,
b1) le traitement thermique en présence de vapeur d'eau de la zéolithe issue de ladite étape a1) de manière à obtenir une zéolithe EU-1 modifiée,
c1) la mise en forme de ladite zéolithe EU-1 modifiée avec une matrice pour former un support zéolithique modifié,
d1) le dépôt d'au moins un métal du groupe VIII de la classification périodique des éléments, l'ordre de réalisation desdites étapes c1) et d1) étant indifférent à la suite de ladite étape b1).

Un deuxième mode de réalisation de la préparation du catalyseur selon l'invention consiste en un procédé comprenant au moins les étapes suivantes :
a2) la synthèse d'au moins une zéolithe EU-1 ayant un rapport atomique Si/Al global compris entre 5 et 100, de préférence entre 10 et 50 et de manière encore plus préférée entre 10 et 35,
b2) la mise en forme de la zéolithe EU-1 issue de ladite étape a2) avec une matrice pour former un support zéolithique,
c2) le traitement thermique en présence de vapeur d'eau du support zéolithique formé à l'étape b2),
d2) le dépôt d'au moins un métal du groupe VIII de la classification périodique des éléments sur le support zéolithique modifié selon l'étape c2).

Un troisième mode de réalisation de la préparation du catalyseur selon l'invention consiste en un procédé comprenant au moins les étapes suivantes :
a3) la synthèse d'au moins une zéolithe EU-1 ayant un rapport atomique Si/Al global compris entre 5 et 100, de préférence entre 10 et 50 et de manière encore plus préférée entre 10 et 35,
b3) la mise en forme de la zéolithe EU-1 issue de ladite étape a3) avec une matrice pour former un support zéolithique,
c3) le dépôt d'au moins un métal du groupe VIII de la classification périodique des éléments sur le support zéolithique issu de ladite étape b3),
d3) le traitement thermique en présence de vapeur d'eau du support zéolithique imprégné d'au moins dudit métal du groupe VIII issu de ladite étape c3).

Conformément à l'invention, la zéolithe EU-1 initiale, n'ayant pas encore été modifiée pour être comprise dans le catalyseur selon l'invention, préparée dans l'étape a1), a2) ou a3), présente un réseau microporeux monodimensionnel, dont le diamètre des pores est de 4,1 x 5,4 Å (1 Å = 1 Angstrôm = 10⁻¹⁰ m) (« Atlas of Zeolite Framework Types », Ch. Baerlocher, W.M. Meier et D.H. Olson, 5^{ème} Edition, 2001). D'autre part, N.A. Briscoe *et al* ont enseigné dans un article de la revue Zeolites (1988, 8, 74) que ces canaux monodimensionnels possèdent des poches latérales de profondeur 8,1 Å et de diamètre 6,8 x 5,8 Å. Les caractéristiques physico-chimiques de ladite zéolithe EU-1 ont déjà été décrites dans le brevet EP-B1- 42 226.

Pour la synthèse de la zéolithe EU-1 selon ladite étape a1), a2) ou a3), l'Homme du métier se référera utilement à l'enseignement du brevet EP-0.042.226 B1 qui décrit la synthèse d'une zéolithe EU-1 brute de synthèse.
Plus précisément, pour la préparation d'une zéolithe EU-1 selon ladite étape a1), a2) ou a3), on mélange en milieu aqueux au moins une source de silicium, au moins une source d'aluminium, au moins un structurant organique azoté Q de formule R₁R₂R₃ - N⁺ - (CH₂)ₙ - N⁺ - R₄R₅R₆, dans laquelle n est compris entre 3 et 12, les groupes R₁ à R₆, identiques ou différents, sont des groupes alkyles ayant de 1 à 8 atomes de carbone, jusqu'à cinq desdits groupes R₁ à R₆ pouvant être de l'hydrogène, éventuellement des germes zéolithiques.
Le mélange réactionnel a la composition molaire suivante :
SiO₂/Al₂O₃: 10 - 150
OH-/SiO₂ : 0,1 - 6
(M⁺ + Q)/Al₂O₃ : 0,5 - 100
Q/(M⁺ + Q) : 0,1 - 10
H₂O/SiO₂ : 1 - 100
Q étant le cation R₁R₂R₃ - N⁺ - (CH₂)ₙ - N⁺ - R₄R₅R₆, décrit ci-dessus, de préférence le 1,6 N,N,N,N',N',N'-hexaméthylhexaméthylène diammonium et M⁺ étant un cation alcalin ou l'ammonium.
Ledit mélange réactionnel est mis à réagir sous la pression autogène, éventuellement avec un apport d'un gaz, par exemple de l'azote, à une température comprise entre 85 et 250°C jusqu'à ce que les cristaux de zéolithes EU-1 se forment. La durée de la réaction est comprise entre 1 minute et plusieurs mois suivant la composition des réactifs, le mode de chauffage et de mélange, la température de la réaction et l'agitation. Au terme de la réaction, la phase solide est collectée sur un filtre et lavée. A ce stade là, la zéolithe EU-1 est dite brute de synthèse et contient dans sa porosité intracristalline au moins le cation R₁R₂R₃ - N⁺ - (CH₂)ₙ - N⁺ - R₄R₅R₆, de préférence le 1,6 N,N,N,N',N',N'-hexaméthylhexaméthylène diammonium. Conformément à l'invention, ladite zéolithe EU-1 brute de synthèse, obtenue à l'issue de l'étape a1), a2) ou a3), présente un rapport atomique Si/Al global compris entre 5 et 100, de préférence entre 10 et 50 et de manière encore plus préférée entre 10 et 35. Le rapport atomique Si/Al global, déterminé par fluorescence X ou absorption atomique, prend en compte aussi bien les atomes d'aluminium présents dans la charpente zéolithique que les atomes d'aluminium éventuellement présents hors de ladite charpente zéolithique encore appelés aluminium extra-réseau. Le pourcentage pondéral des atomes d'aluminium tétracoordinés présents dans la zéolithe EU-1 brute de synthèse préparée selon ladite étape a1), a2) ou a3), déterminé par analyse RMN-MAS de l'aluminium comme décrit plus haut dans la présente description, est supérieur à 90%, de préférence supérieur à 95%, de manière très préférée supérieur à 98% et de manière encore plus préférée il est égal à 100%.

Conformément au premier mode de réalisation de la préparation du catalyseur selon l'invention, la zéolithe EU-1 brute de synthèse issue de ladite étape a1) est soumise à une calcination sous flux d'air sec, à une température comprise entre 400 et 600°C puis est soumise à au moins un traitement thermique en présence de vapeur d'eau selon ladite étape b1). La durée de la calcination est variable et est comprise entre plusieurs heures et plusieurs jours. Le traitement par calcination, préalable à ladite étape b1), de ladite zéolithe EU-1 issue de ladite étape a1) a pour but d'éliminer le structurant organique présent dans la microporosité de la zéolithe, par exemple le cation R₁R₂R₃ - N⁺ - (CH₂)ₙ - N⁺ - R₄R₅R₆, de préférence le 1,6 N,N,N,N',N',N'-hexaméthylhexaméthylène diammonium. De manière préférée, un ou plusieurs échange(s) ionique(s) par au moins une solution NH₄NO₃ est(sont) opéré(s) entre la calcination sous flux d'air sec et le traitement thermique en présence de vapeur d'eau de manière à éliminer au moins en partie, de préférence pratiquement totalement, le cation alcalin, en particulier le sodium, éventuellement présent en position cationique dans la zéolithe sous sa forme brute de synthèse. Chaque échange est réalisé à une température préférentiellement comprise entre 50 et 150°C pendant une durée avantageusement comprise entre 2 heures et 10 heures. On utilise généralement une solution aqueuse de nitrate d'ammonium NH₄NO₃ ayant une normalité comprise entre 7 et 12N. De même, à la fin de la mise en oeuvre de ladite étape b1) de traitement thermique, il est possible de réaliser un ou plusieurs échange(s) ionique(s) par au moins une solution NH₄NO₃, de manière à éliminer les cations alcalins résiduels et en particulier le sodium.

La zéolithe EU-1 issue de ladite étape a1), puis calcinée et préférentiellement échangée de manière à ce qu'elle se trouve sous sa forme NH₄, est soumise à au moins un traitement thermique en présence de vapeur d'eau selon l'étape b1) du premier mode de réalisation de la préparation du catalyseur selon l'invention. Les conditions opératoires du traitement thermique en présence de vapeur d'eau, en particulier la température et la durée dudit traitement ainsi que le pourcentage volumique de vapeur d'eau, sont adaptées de manière à obtenir une zéolithe EU-1 modifiée dont le nombre d'atomes d'aluminium hexacoordinés représente plus de 20% poids du nombre total d'atomes d'aluminium présents dans ladite zéolithe modifiée. De manière avantageuse, le traitement thermique en présence de vapeur d'eau est réalisé à une température comprise entre 200 et 470°C, de préférence entre 320 et 460°C. La durée dudit traitement thermique est généralement supérieure ou égale à 0,5 heure, préférentiellement comprise entre 0,5 heure et 24 heures, et très préférentiellement comprise entre 1 heure et 12 heures. Le pourcentage volumique de vapeur d'eau durant le traitement thermique est généralement compris entre 5 et 100%, de préférence entre 20 et 100%, de manière encore plus préférée entre 40% et 100%. La fraction volumique autre que la vapeur d'eau éventuellement présente est formée d'air. Le débit de gaz formé de vapeur d'eau et éventuellement d'air est avantageusement compris entre 0,2 1/h/g de zéolithe et 10 1/h/g de zéolithe. Le traitement thermique en présence de vapeur d'eau peut être répété autant de fois qu'il est nécessaire pour obtenir la zéolithe EU-1 modifiée possédant les caractéristiques désirées en particulier un nombre d'atomes d'aluminium hexacoordinés représentant plus de 20% poids du nombre total d'atomes d'aluminium présents dans ladite zéolithe. Le nombre de traitement thermique selon ladite étape b1) est préférentiellement inférieur à 4 et on réalise avantageusement un seul traitement thermique selon ladite étape b1).

La préparation du catalyseur selon le premier mode de réalisation se poursuit par la mise en oeuvre de ladite étape c1) de mise en forme et par la mise en oeuvre de ladite étape d1) de dépôt d'au moins un métal du groupe VIII de la classification périodique des éléments. L'ordre de réalisation desdites étapes c1) et d1), subséquentes à ladite étape b1), est indifférent. De préférence, ladite étape c1) précède ladite étape d1).

Pour la mise en oeuvre de ladite étape c1) de mise en forme de ladite zéolithe EU-1 modifiée, on utilise une matrice choisie parmi les argiles, la magnésie, les alumines, les silices, l'oxyde de titane, l'oxyde de bore, la zircone, les phosphates d'aluminium, les phosphates de titane, les phosphates de zirconium, les silices-alumines et le charbon ou un mélange d'au moins deux de ces compositions. De préférence, la matrice est une alumine. Avantageusement, la zéolithe EU-1 modifiée associée à la matrice est mise sous forme de billes ou d'extrudés, très avantageusement sous forme d'extrudés. L'ensemble zéolithe EU-1 modifiée - matrice constitue le support zéolithique modifié du catalyseur selon l'invention.
La mise en forme conformément à ladite étape c1) consiste plus particulièrement à malaxer la zéolithe EU-1 modifiée, dans un gel humide de matrice, préférentiellement d'alumine, obtenu généralement par mélange d'au moins un acide et d'une poudre de matrice, pendant une durée nécessaire à l'obtention d'une bonne homogénéité de la pâte, soit par exemple pendant une dizaine de minutes, puis à passer la pâte ainsi obtenue à travers une filière pour former des extrudés, par exemple de diamètre de 0,4 à 4 mm. La mise en forme est généralement suivie d'un séchage puis d'une calcination. Le séchage est avantageusement réalisé à une température comprise entre 100 et 150°C pendant une durée comprise entre 5 et 20 heures en étuve. La calcination est avantageusement réalisée à une température comprise entre 250°C et 600°C pendant une durée comprise entre 1 et 8 heures.

L'étape d1) de préparation du catalyseur comprenant une zéolithe EU-1 modifiée, consiste à introduire au moins un métal du groupe VIII de la classification périodique des éléments et éventuellement au moins un métal choisi parmi les métaux des groupes IIIA, IVA et VIIB.
Ledit métal du groupe VIII présent dans le catalyseur selon l'invention est choisi parmi le fer, le cobalt, le nickel, le ruthénium, le rhodium, le palladium, l'osmium, l'iridium et le platine, de préférence parmi les métaux nobles et très préférentiellement parmi le palladium et le platine. De manière encore plus préférée, ledit métal du groupe VIII est le platine. Selon la méthode mise en oeuvre pour le dépôt dudit métal du groupe VIII, comme il est indiqué ci-après dans la description, ledit métal du groupe VIII, de préférence le platine, peut être déposé de manière prépondérante sur la zéolithe modifiée ou sur la matrice.
Ledit métal choisi parmi les métaux des groupes IIIA, IVA et VIIB et éventuellement présent dans le catalyseur selon l'invention est choisi parmi le gallium, l'indium, l'étain et le rhénium, de préférence parmi l'indium, l'étain et le rhénium.
La préparation du catalyseur selon l'invention, après l'obtention de la zéolithe EU-1 modifiée et de sa mise en forme, peut être effectuée par toute méthode connue de l'Homme du métier. De manière préférée, à la suite de la calcination réalisée à la fin de l'étape c1) de mise en forme, au moins un métal du groupe VIII est introduit sur le support zéolithique modifié, à savoir soit majoritairement sur la matrice, soit majoritairement sur la zéolithe modifiée soit encore sur l'ensemble zéolithe modifiée-matrice. Le dépôt dudit métal sur le support zéolithique est avantageusement effectué par la technique d'imprégnation à sec, la technique d'imprégnation par excès ou par échange ionique. Lorsque plusieurs métaux sont introduits, ceux-ci peuvent être introduits soit tous de la même façon soit par des techniques différentes.
Tous les précurseurs de métaux du groupe VIII conviennent pour le dépôt d'un ou de plusieurs métal(ux) du groupe VIII sur le support zéolithique modifié. En particulier, pour tout métal noble du groupe VIII, on peut utiliser des composés ammoniaqués ou des composés tels que par exemple le chloroplatinate d'ammonium, le dichlorure de platine dicarbonyle, l'acide hexahydroxyplatinique, le chlorure de palladium ou le nitrate de palladium. Le platine est généralement introduit sous forme d'acide hexachloroplatinique. L'introduction du métal noble du groupe VIII est de préférence effectuée par imprégnation à l'aide d'une solution aqueuse ou organique de l'un des composés métalliques cités ci-dessus. Parmi les solvants organiques utilisables, on peut citer les hydrocarbures paraffiniques, naphténiques ou aromatiques contenant par exemple de 6 à 12 atomes de carbone par molécule, et les composés organiques halogénés contenant par exemple de 1 à 12 atomes de carbone par molécule. On peut citer par exemple le n-heptane, le méthylcyclohexane, le toluène et le chloroforme. On peut aussi utiliser les mélanges de solvants.
Le contrôle de certains paramètres mis en oeuvre lors du dépôt, en particulier la nature du précurseur du (des) métal(ux) du groupe VIII utilisé(s), permet d'orienter le dépôt du(es)dit(s) métal(ux) majoritairement sur la matrice ou sur la zéolithe modifiée.
Ainsi, pour introduire le(s) métal(ux) du groupe VIII, préférentiellement le platine et/ou le palladium, majoritairement sur la matrice, on peut mettre en oeuvre un échange anionique avec de l'acide hexachloroplatinique et/ou de l'acide hexachloropalladique, en présence d'un agent compétiteur, par exemple de l'acide chlorhydrique, le dépôt étant en général suivi d'une calcination, par exemple à une température comprise entre 350 et 550°C et pendant une durée comprise entre 1 et 4 heures. Avec de tels précurseurs, le(s) métal(ux) du groupe VIII est(sont) déposé(s) majoritairement sur la matrice et le(s)dit(s) métal(ux) présente(nt) une bonne dispersion et une bonne répartition macroscopique à travers le grain de catalyseur.
On peut aussi envisager de déposer le(s) métal(ux) du groupe VIII, préférentiellement le platine et/ou le palladium, par échange cationique de manière à ce que le(s)dit(s) métal(ux) soi(en)t déposé(s) majoritairement sur la zéolithe modifiée. Ainsi, dans le cas du platine, le précurseur peut être par exemple choisi parmi :
- les composés ammoniaqués tels que les sels de platine (II) tétrammines de formule Pt(NH₃)₄X₂, les sels de platine (IV) hexammines de formule Pt(NH₃)₆X₄ ; les sels de platine (IV) halogénopentammines de formule (PtX(NH₃)₅)X₃ ; les sels de platine N-tétrahalogénodiammines de formule PtX₄(NH₃)₂ ; et
- les composés halogénés de formule H(Pt(acac)₂X) ;
X étant un halogène choisi dans le groupe formé par le chlore, le fluor, le brome et l'iode, X étant de préférence le chlore, et "acac" représentant le groupe acétylacétonate (de formule brute C₅H₇O₂), dérivé de l'acétylacétone. Avec de tels précurseurs, le(s) métal(ux) du groupe VIII est(sont) déposé(s) majoritairement sur la zéolithe et le(s)dit(s) métal(ux) présente(nt) une bonne dispersion et une bonne répartition macroscopique à travers le grain de catalyseur.
L'imprégnation à sec du métal du groupe VIII sur le support zéolithique conduit au dépôt dudit métal à la fois sur la matrice et sur la zéolithe modifiée.
Dans le cas où le catalyseur de l'invention contient également au moins un métal choisi parmi les métaux des groupes IIIA, IVA et VIIB, toutes les techniques de dépôt d'un tel métal connues de l'homme du métier et tous les précurseurs de tels métaux peuvent convenir.
On peut ajouter le(s) métal(ux) du groupe VIII et celui(ceux) des groupes IIIA, IVA et VIIB, soit séparément soit simultanément dans au moins une étape unitaire. Lorsqu'au moins un métal des groupes IIIA, IVA et VIIB est ajouté séparément, il est préférable qu'il soit ajouté après le métal du groupe VIII.
Le métal additionnel choisi parmi les métaux des groupes IIIA, IVA et VIIB peut être introduit par l'intermédiaire de composés tels que par exemple les chlorures, les bromures et les nitrates des métaux des groupes IIIA, IVA et VIIB. Par exemple dans le cas de l'indium, on utilise avantageusement le nitrate ou le chlorure et dans le cas du rhénium, on utilise avantageusement l'acide perrhénique. Le métal additionnel choisi parmi les métaux des groupes IIIA, IVA et VIIB peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles métaux. Dans ce dernier cas, l'introduction du métal est avantageusement effectuée à l'aide d'une solution du composé organométallique dudit métal dans un solvant organique. On peut également employer des composés organohalogénés du métal. Comme composés organiques de métaux, on peut citer en particulier le tétrabutylétain, dans le cas de l'étain, et le triphénylindium, dans le cas de l'indium.

Si le métal additionnel choisi parmi les métaux des groupes IIIA, IVA et VIIB est introduit avant le métal du groupe VIII, le composé du métal IIIA, IVA et/ou VIIB utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain. Dans ce cas, avant de procéder à l'introduction d'au moins un métal du groupe VIII, on procédera à une calcination sous air.
De plus, des traitements intermédiaires tels que par exemple une calcination et/ou une réduction peuvent être appliqués entre les dépôts successifs des différents métaux.
La préparation du catalyseur selon ledit premier mode de réalisation se termine généralement par une calcination, habituellement à une température comprise entre 250°C et 600°C, pour une durée comprise entre 0,5 et 10 heures, de préférence précédée d'un séchage, par exemple à l'étuve, à une température allant de la température ambiante à 250°C, de préférence de 40°C à 200°C. Ladite étape de séchage est de préférence menée pendant la montée en température nécessaire pour effectuer ladite calcination. On peut mettre en oeuvre une réduction préalable du catalyseur *ex situ*, sous courant d'hydrogène, par exemple à une température de 450°C à 600°C, pendant une durée de 0,5 à 4 heures.

Conformément au deuxième mode de réalisation de la préparation du catalyseur selon l'invention, la zéolithe EU-1 brute de synthèse obtenue à l'étape a2) est ensuite mise en forme avec une matrice de manière à préparer un support zéolithique selon ladite étape b2). La zéolite EU-1 brute de synthèse est préférentiellement calcinée puis échangée avant d'être mise en forme avec une matrice, préférentiellement une alumine. Toutefois, la mise en forme selon l'étape b2) peut être réalisée directement en utilisant la zéolithe EU-1 brute de synthèse, la calcination puis l'échange ionique étant réalisés sur la zéolithe mise en forme. La calcination de la zéolithe brute de synthèse est réalisée sous flux d'air sec, à une température comprise entre 400 et 600°C. La durée de la calcination est variable et est comprise entre plusieurs heures et plusieurs jours. Le traitement par calcination, préalable ou subséquent à ladite étape b2), de ladite zéolithe EU-1 issue de ladite étape a2) a pour but d'éliminer le structurant organique présent dans la microporosité de la zéolithe, par exemple le cation R₁R₂R₃ - N⁺ - (CH₂)ₙ - N⁺ - R₄R₅R₆, de préférence le 1,6 N,N,N,N',N',N'-hexaméthylhexaméthylène diammonium. Un ou plusieurs échange(s) ionique(s) par au moins une solution NH₄NO₃ est(sont) opéré(s) à la suite de la calcination sous flux d'air sec de manière à éliminer au moins en partie, de préférence pratiquement totalement, le cation alcalin, en particulier le sodium, éventuellement présent en position cationique dans la zéolithe sous sa forme brute de synthèse. Chaque échange est réalisé à une température préférentiellement comprise entre 50 et 150°C pendant une durée avantageusement comprise entre 2 heures et 10 heures. On utilise généralement une solution aqueuse de nitrate d'ammonium NH₄NO₃ ayant une normalité comprise entre 7 et 12N.
La matrice employée pour la mise en oeuvre de ladite étape b2) est choisie parmi celles décrites pour la mise en oeuvre de l'étape c1) du premier mode de réalisation de la préparation du catalyseur selon l'invention. De même, la mise en oeuvre de ladite étape b2) est réalisée selon un même protocole (malaxage, extrusion, séchage puis calcination) et dans des conditions opératoires analogues à celles décrites pour la mise en oeuvre de l'étape c1) du premier mode de réalisation de la préparation du catalyseur selon l'invention. Avantageusement, la zéolithe EU-1 associée à la matrice est mise sous forme de billes ou d'extrudés, préférentiellement sous forme d'extrudés, et constitue le support zéolithique du catalyseur de l'invention.

Le support zéolithique obtenu à l'issue de ladite étape b2) contient une zéolithe EU-1 dont la charpente zéolithique n'a pas encore été modifiée. Ledit support est ensuite soumis à au moins un traitement thermique réalisé en présence de vapeur d'eau selon ladite étape c2) de manière à ce que la zéolithe EU-1 comprise dans ledit support zéolithique présente un nombre d'atomes d'aluminium hexacoordinés représentant plus de 20% poids, préférentiellement plus de 22% poids, de manière plus préférée plus de 25% poids et de manière encore plus préférée plus de 30% poids, du nombre total d'atomes d'aluminium présents dans ladite zéolithe.

De manière avantageuse, le traitement thermique en présence de vapeur d'eau auquel est soumis le support zéolithique est réalisé à une température comprise entre 200 et 470°C, de préférence entre 320 et 460°C. La durée dudit traitement thermique est généralement supérieure ou égale à 0,5 heure, préférentiellement comprise entre 0,5 heure et 24 heures, et très préférentiellement comprise entre 1 heure et 12 heures. Le pourcentage volumique de vapeur d'eau durant le traitement thermique est généralement compris entre 5 et 100%, de préférence entre 20 et 100%, de manière encore plus préférée entre 40% et 100%. La fraction volumique autre que la vapeur d'eau éventuellement présente est formée d'air. Le débit de gaz formé de vapeur d'eau et éventuellement d'air est compris entre 0,2 l/h/g de support zéolithique et 10 l/h/g de support zéolithique. Le traitement thermique en présence de vapeur d'eau peut être répété autant de fois qu'il est nécessaire pour obtenir la zéolithe EU-1 modifiée possédant les caractéristiques désirées en particulier un nombre d'atomes d'aluminium hexacoordinés représentant plus de 20% poids du nombre total d'atomes d'aluminium présents dans ladite zéolithe. Le nombre de traitement thermique selon ladite étape c2) est préférentiellement inférieur à 4 et on réalise avantageusement un seul traitement thermique selon ladite étape c2).

Conformément au deuxième mode de réalisation de la préparation du catalyseur selon l'invention, la zéolithe EU-1 comprise dans le support zéolithique obtenu à l'issue de ladite étape c2) est modifiée. La préparation du catalyseur se poursuit par le dépôt d'au moins un métal du groupe VIII et éventuellement d'au moins un métal choisi parmi les métaux des groupes IIIA, IVA, VIIB de la classification périodique des éléments sur ledit support zéolithique selon ladite étape d2). Le(s) métal(ux) du groupe VIII et éventuellement le(s) métal(ux) choisi(s) parmi les métaux des groupes IIIA, IVA et VIIB est(sont) choisi(s) parmi la(les) liste(s) de métaux décrites ci-dessus pour la mise en oeuvre de ladite étape d1) du premier mode de réalisation de la préparation du catalyseur de l'invention. Les précurseurs du(des) métal(ux) du groupe VIII sont analogues à ceux décrits ci-dessus pour la mise en oeuvre de ladite étape d1). Le(s)dit(s) métal(ux) du groupe VIII est(sont) introduit(s) sur le support zéolithique, à savoir soit majoritairement sur la zéolithe modifiée par échange cationique, soit majoritairement sur la matrice par échange anionique soit encore sur l'ensemble zéolithe modifiée-matrice par imprégnation à sec. Les protocoles de réalisation de l'une ou l'autre de ces techniques sont analogues à ceux décrits ci-dessus pour la mise en oeuvre de ladite étape d1) du premier mode de réalisation de la préparation du catalyseur de l'invention. Lorsque le catalyseur comprend au moins un métal choisi parmi les métaux des groupes IIIA, IVA et VIIB, les techniques d'introduction de ces métaux ainsi que les précurseurs desdits métaux des groupes IIIA, IVA et VIIB sont celles et ceux déjà décrit(e)s ci-dessus pour la mise en oeuvre de ladite étape d1) du premier mode de réalisation de la préparation du catalyseur de l'invention.
La préparation du catalyseur selon ledit deuxième mode de réalisation décrit ci-dessus se termine généralement par une calcination, habituellement à une température comprise entre 250°C et 600°C, pour une durée comprise entre 0,5 et 10 heures, de préférence précédée d'un séchage, par exemple à l'étuve, à une température allant de la température ambiante à 250°C, de préférence de 40°C à 200°C. Ladite étape de séchage est de préférence menée pendant la montée en température nécessaire pour effectuer ladite calcination. On peut mettre en oeuvre une réduction préalable du catalyseur *ex situ*, sous courant d'hydrogène, par exemple à une température de 450°C à 600°C, pendant une durée de 0,5 à 4 heures.

Conformément au troisième mode de réalisation de la préparation du catalyseur selon l'invention, la zéolithe EU-1 brute de synthèse obtenue à l'étape a3) est ensuite mise en forme avec une matrice de manière à préparer un support zéolithique selon ladite étape b3). La zéolite EU-1 brute de synthèse est préférentiellement calcinée puis échangée avant d'être mise en forme avec une matrice, préférentiellement une alumine. Toutefois, la mise en forme selon l'étape b3) peut être réalisée directement en utilisant la zéolithe EU-1 brute de synthèse, la calcination puis l'échange ionique étant réalisés sur la zéolithe mise en forme comme cela a été décrit pour la mise en oeuvre du deuxième mode de réalisation de la préparation du catalyseur selon l'invention. La calcination réalisée en vue d'éliminer le structurant organique présent dans la microporosité de la zéolithe EU-1 préparée à l'étape a3) puis l'échange ionique sont effectués dans des conditions analogues à celles décrites pour la mise en oeuvre dudit premier mode de réalisation de la préparation du catalyseur de l'invention. La matrice utilisée, préférentiellement une alumine, et le protocole de réalisation de la mise en forme (malaxage, extrusion, séchage et calcination) sont analogues à ce qui a été décrit pour la mise en oeuvre de l'étape c1) du premier mode de réalisation de la préparation du catalyseur selon l'invention. Avantageusement, la zéolithe EU-1 associée à la matrice est mise sous forme de billes ou d'extrudés, très avantageusement sous forme d'extrudés, et constitue le support zéolithique du catalyseur. La préparation du catalyseur selon le troisième mode de réalisation de l'invention se poursuit par le dépôt d'au moins un métal du groupe VIII et éventuellement d'au moins un métal choisi parmi les métaux des groupes IIIA, IVA et VIIB de la classification périodique des éléments sur ledit support zéolithique selon ladite étape c3). Le(s) métal(ux) du groupe VIII et éventuellement le(s) métal(ux) choisi(s) parmi les métaux des groupes IIIA, IVA et VIIB est(sont) choisi(s) parmi la(les) liste(s) de métaux décrites ci-dessus pour la mise en oeuvre de ladite étape d1) du premier mode de réalisation de la préparation du catalyseur de l'invention. De préférence, le métal du groupe VIII est le platine. Les précurseurs du(des) métal(ux) du groupe VIII sont analogues à ceux décrits ci-dessus pour la mise en oeuvre de ladite étape d1). Le(s)dit(s) métal(ux) du groupe VIII est(sont) introduit(s) sur le support zéolithique, à savoir soit majoritairement sur la zéolithe par échange cationique, soit majoritairement sur la matrice par échange anionique soit encore sur l'ensemble zéolithe-matrice par imprégnation à sec. Les protocoles de réalisation de l'une ou l'autre de ces techniques de dépôt sont analogues à ceux décrits ci-dessus pour la mise en oeuvre de ladite étape d1) du premier mode de réalisation de la préparation du catalyseur de l'invention. Lorsque le catalyseur comprend au moins un métal choisi parmi les métaux des groupes IIIA, IVA et VIIB, les techniques d'introduction de ces métaux ainsi que les précurseurs desdits métaux des groupes IIIA, IVA et VIIB sont celles et ceux déjà décrit(e)s ci-dessus pour la mise en oeuvre de ladite étape d1) du premier mode de réalisation de la préparation du catalyseur de l'invention. Le catalyseur ainsi obtenu est calciné, habituellement à une température comprise entre 250°C et 600°C, pour une durée comprise entre 0,5 et 10 heures, après avoir été préférentiellement séché, par exemple à l'étuve, à une température allant de la température ambiante à 250°C, de préférence de 40°C à 200°C. Ladite étape de séchage est de préférence menée pendant la montée en température nécessaire pour effectuer ladite calcination. On peut éventuellement mettre en oeuvre une réduction préalable du catalyseur *ex situ,* sous courant d'hydrogène, par exemple à une température de 450°C à 600°C, pendant une durée de 0,5 à 4 heures, ladite étape de réduction étant réalisée à la suite de la calcination du catalyseur obtenu à l'étape c3).
A l'issue de ladite étape c3), le catalyseur préparé selon ledit troisième mode de réalisation comprend au moins une matrice, préférentiellement de l'alumine, au moins un métal du groupe VIII, préférentiellement le platine, et au moins une zéolithe EU-1. La modification de la charpente zéolithique de la zéolithe EU-1 de manière à ce que les atomes d'aluminium hexacoordinés extra-réseau représentent plus de 20% poids du nombre total des atomes d'aluminium présents dans la zéolithe EU-1 comprise dans le catalyseur final est réalisée au cours d'une étape d3) laquelle consiste à soumettre le catalyseur obtenu à l'issue de ladite étape c3) à un traitement thermique en présence de vapeur d'eau. De manière avantageuse, le traitement thermique en présence de vapeur d'eau est réalisé à une température comprise entre 200 et 470°C, de préférence entre 320 et 460°C. La durée dudit traitement thermique est généralement supérieure ou égale à 0,5 heure, préférentiellement comprise entre 0,5 heure et 24 heures, et très préférentiellement comprise entre 1 heure et 12 heures. Le pourcentage volumique de vapeur d'eau durant le traitement thermique est généralement compris entre 5 et 100%, de préférence entre 20 et 100%, de manière encore plus préférée entre 40% et 100%. La fraction volumique autre que la vapeur d'eau éventuellement présente est formée d'air. Le débit de gaz formé de vapeur d'eau et éventuellement d'air est compris entre 0,2 l/h/g de solide et 10 l/h/g de solide. Le traitement thermique en présence de vapeur d'eau peut être répété autant de fois qu'il est nécessaire pour obtenir la zéolithe EU-1 modifiée possédant les caractéristiques désirées, en particulier un nombre d'atomes d'aluminium hexacoordinés représentant plus de 20% poids du nombre total d'atomes d'aluminium présents dans ladite zéolithe. Le nombre de traitement thermique selon ladite étape d3) est préférentiellement inférieur à 4 et on réalise avantageusement un seul traitement thermique selon ladite étape d3).

Pour la préparation du catalyseur selon l'invention, ledit premier mode de réalisation et ledit troisième mode de réalisation sont préférés. De manière très préférée, le catalyseur selon l'invention est réalisé en mettant en oeuvre ledit troisième mode de réalisation. Ledit troisième mode de réalisation de la préparation du catalyseur selon l'invention est particulièrement avantageux lorsque l'étape d3) de traitement thermique est réalisée *in situ*, c'est-à-dire lorsque le catalyseur obtenu à l'issue de ladite étape c3) est introduit dans un réacteur catalytique réalisant la transformation d'une charge hydrocarbonée, en particulier dans un réacteur réalisant l'isomérisation d'une charge aromatique contenant au moins un composé aromatique ayant huit atomes de carbone par molécule. La zéolithe EU-1 comprise dans le catalyseur de l'invention est alors modifiée *in situ*. Ladite étape d3), lorsqu'elle est réalisée *in situ*, est généralement suivie d'un séchage du catalyseur à une température préférentiellement comprise entre 100 et 300°C, lequel est préférentiellement suivi d'une réduction sous courant d'hydrogène, elle-même avantageusement suivie d'une sulfuration réalisée dans les conditions décrites ci-dessous.

Quel que soit le mode de réalisation de la préparation du catalyseur selon l'invention, on peut mettre en oeuvre une réduction préalable du catalyseur final *ex situ,* sous courant d'hydrogène, par exemple à une température de 450°C à 600°C, pendant une durée de 0,5 à 4 heures.

Dans le cas où le catalyseur ne contient pas de soufre, une réduction du métal sous hydrogène est réalisée *in situ* avant injection de la charge.

Dans le cas où le catalyseur de l'invention contient du soufre, le soufre est introduit sur le catalyseur mis en forme, calciné, contenant le ou les métaux cités précédemment, soit *in situ* avant la réaction catalytique, soit *ex situ.* La sulfuration éventuelle intervient après la réduction. Dans le cas d'une sulfuration *in situ*, la réduction, si le catalyseur n'a pas été préalablement réduit, intervient avant la sulfuration. Dans le cas d'une sulfuration *ex situ*, on effectue la réduction puis la sulfuration. La sulfuration s'effectue en présence d'hydrogène en utilisant tout agent sulfurant bien connu de l'homme de métier, tel que par exemple le sulfure de diméthyle ou le sulfure d'hydrogène. Par exemple, le catalyseur est traité avec une charge contenant du sulfure de diméthyle en présence d'hydrogène, avec une concentration telle que le rapport atomique soufre/métal soit de 1,5. Le catalyseur est ensuite maintenu pendant environ 3 heures à environ 400°C sous débit d'hydrogène avant l'injection de la charge.

La présente invention a également pour objet un procédé d'isomérisation d'une coupe contenant au moins un composé aromatique à huit atomes de carbone par molécule, ledit procédé comprenant la mise en contact de ladite coupe aromatique avec au moins ledit catalyseur selon l'invention présent dans un réacteur catalytique. Ladite coupe aromatique contenant au moins un composé aromatique ayant huit atomes de carbone par molécule comprend en particulier comme composé aromatique ayant huit atomes de carbone par molécule soit uniquement un mélange de xylènes, soit uniquement de l'éthylbenzène, soit un mélange de xylène(s) et d'éthylbenzène. Ledit procédé d'isomérisation est mis en oeuvre généralement selon les conditions opératoires suivantes :
- une température de 300°C à 500°C, de préférence de 320°C à 450°C et de manière encore plus préférée de 340°C à 430°C ;
- une pression partielle d'hydrogène de 0,3 à 1,5 MPa, de préférence de 0,4 et 1,2 MPa et de manière encore préférée de 0,7 à 1,2 MPa ;
- une pression totale de 0,45 à 1,9 MPa, de préférence de 0,6 à 1,5 MPa ; et
- une vitesse spatiale d'alimentation, exprimée en kilogramme de charge introduite par kilogramme de catalyseur et par heure, de 0,25 à 30 h⁻¹, de préférence de 1 à 10 h⁻¹ et de manière encore préférée de 2 à 6 h⁻¹.

Conformément au procédé d'isomérisation selon l'invention, le catalyseur utilisé pour la mise en oeuvre dudit procédé peut être introduit dans le réacteur catalytique soit lorsque la zéolithe EU-1 comprise dans ledit catalyseur est déjà modifiée au sens de la présente invention soit lorsqu'elle ne l'est pas encore. Dans ce dernier, la modification de la charpente zéolithique de la zéolithe EU-1 intervient une fois que le catalyseur comprenant au moins une matrice, de préférence de l'alumine, au moins un métal du groupe VIII, de préférence du platine, et au moins une zéolithe EU-1 dont plus de 90% poids des atomes d'aluminium sont des atomes d'aluminium tétracoordinés est introduit dans le réacteur catalytique. Le traitement thermique en présence de vapeur d'eau, effectué pour la modification de la zéolithe EU-1 présente dans le catalyseur chargé dans le réacteur, est réalisé préalablement à la mise en contact du catalyseur de l'invention avec la coupe aromatique à isomériser. Le traitement thermique en présence de vapeur d'eau est ainsi réalisé *in situ* en faisant passer dans le réacteur catalytique un courant de vapeur d'eau, éventuellement additionné d'air, sur le catalyseur y présent et comprenant une zéolithe EU-1 encore non modifiée. La zéolithe EU-1 présente dans le catalyseur a été modifiée par un traitement thermique en présence de vapeur d'eau, ledit traitement étant réalisé *in-situ* et préalablement à la mise en contact du catalyseur de l'invention avec la coupe aromatique à isomériser. Ledit traitement thermique en présence de vapeur d'eau, lorsqu'il est réalisé *in situ*, est réalisé dans les mêmes conditions opératoires que celles déjà décrites plus haut pour la mise en oeuvre du traitement thermique en présence de vapeur d'eau dans le premier mode de réalisation de préparation du catalyseur, le deuxième mode de réalisation de préparation du catalyseur ou le troisième mode de réalisation lorsque ledit traitement est réalisé *ex situ.* Ledit traitement thermique en présence de vapeur d'eau réalisé in situ est préférentiellement suivi d'un séchage du catalyseur à une température préférentiellement comprise entre 100 et 300°C, lequel est préférentiellement suivi d'une réduction sous courant d'hydrogène, elle-même éventuellement suivie d'une sulfuration

Les exemples suivants illustrent l'invention sans toutefois en limiter la portée.

Pour les analyses RMN-MAS permettant de repérer et quantifier les atomes d'aluminium tétracoordinés et les atomes d'aluminium hexacoordinés, on utilise un spectromètre Brücker de type Avance 400 MHz à l'aide d'une sonde 4 mm optimisée pour l' ²⁷Al. La vitesse de rotation de chaque échantillon analysé est voisine de 14 kHz. L'atome d'aluminium est un noyau quadripolaire dont le spin est égal à 5/2. Les conditions d'analyse sont les suivantes : champs de radiofréquence égal à 30 kHz, angle d'impulsion égal à π/2 et saturation en eau de l'échantillon analysé. Chaque spectre RMN-MAS est calé en déplacement chimique par rapport à une solution 1 M de nitrate d'aluminium pour laquelle le signal d'aluminium est à zéro ppm.

### Exemple 1 (non conforme) : Préparation d'une zéolithe EU-1

La matière première utilisée est une zéolithe EU-1, brute de synthèse, comprenant le structurant organique à savoir le 1,6 N,N,N,N',N',N'-hexaméthylhexaméthylène diammonium et qui possède un rapport atomique Si/Al global égal à 15,3, une teneur pondérale en sodium correspondant à un rapport atomique Na/Al (en %) égal à 30,8. Cette zéolithe EU-1 a été synthétisée conformément à l'enseignement du brevet EP-B1-0.042.226. Pour la préparation d'une telle zéolithe, le mélange réactionnel présente la composition molaire suivante : 60 SiO₂ : 10,6 Na₂O : 5,27 NaBr : 1,5 Al₂O₃ : 19,5 Hexa-Br₂ : 2777 H₂O. Hexa-Br₂ étant le 1,6 N,N,N,N',N',N'-hexaméthylhexaméthylène diammonium, le brome étant le contre-ion. Le mélange réactionnel est placé dans un autoclave sous agitation (300 tours/min) pendant 5 jours à 180°C.

Cette zéolithe EU-1 subit tout d'abord une calcination dite sèche à 550°C sous flux d'air sec durant 10 heures de manière à éliminer le structurant organique. Puis le solide obtenu est soumis à quatre échanges ioniques dans une solution de NH₄NO₃ 10N, à environ 100°C pendant 4 heures pour chaque échange. Le solide ainsi obtenu est référencé EU-1(1) et possède un rapport Si/Al = 15,3 et un rapport Na/Al=0,51 %.

L'analyse RMN-MAS de cette zéolithe permet de calculer le pourcentage pondéral des atomes d'aluminium tétracoordinés présent dans ladite zéolithe EU-1(1) : il est égal à 100%. Aucun atome d'aluminium hexacoordiné n'est révélé sur le spectre RMN-MAS._

### Exemple 2 (invention) : Préparation d'une zéolithe EU-1 modifiée

La zéolithe EU-1(1) obtenue à l'exemple 1 est placée dans un four où elle est soumise à un traitement hydrothermique : un courant gazeux constitué de 50% volume de vapeur d'eau et 50% volume d'air balaye ladite zéolithe à 450°C durant 2 heures. Le débit de gaz est égal à 1 1/h/g de zéolithe.

A l'issue de ce traitement hydrothermique, on obtient une zéolithe EU-1 modifiée, notée EU-1(2) qui se trouve sous sa forme hydrogène. Ladite zéolithe EU-1(2) possède un rapport atomique Si/Al global égal à celui de la zéolithe EU-1(1), à savoir égal à 15,3. Ladite zéolithe EU-1(2) est analysée par RMN-MAS : le spectre RMN-MAS montre que 23% poids des atomes d'aluminiums présents dans la zéolithe modifiée EU-1 (2) sont des atomes d'aluminium hexacoordinés et que 77% poids des atomes d'aluminium présents dans la zéolithe modifiée EU-1 (2) sont des atomes d'aluminium tétracoordinés.

### Exemple 3 (invention) : Préparation d'une zéolithe EU-1 modifiée

La zéolithe EU-1(1) obtenue à l'exemple 1 est placée dans un four où elle est soumise à un traitement hydrothermique : un courant gazeux constitué de 50% volume de vapeur d'eau et 50% volume d'air balaye ladite zéolithe à 350°C durant 8 heures. Le débit de vapeur d'eau est égal à 1 l/h/g de zéolithe.

A l'issue de ce traitement hydrothermique, on obtient une zéolithe EU-1 modifiée, notée EU-1(3), qui se trouve sous sa forme hydrogène. Ladite zéolithe EU-1(3) possède un rapport atomique Si/Al global égal à celui de la zéolithe EU-1(1), à savoir égal à 15,3. Ladite zéolithe EU-1(3) est analysée par RMN-MAS : le spectre RMN-MAS montre que 46% poids des atomes d'aluminiums présents dans la zéolithe modifiée EU-1(3) sont des atomes d'aluminium hexacoordinés et que 54% poids des atomes d'aluminium présents dans la zéolithe modifiée EU-1(3) sont des atomes d'aluminium tétracoordinés.

### Exemple 4 (non conforme) : Préparation du catalyseur A comprenant une zéolithe EU-1 non modifiée

La zéolithe EU-1(1) obtenue à l'exemple 1 est ensuite mise en forme par extrusion avec un gel d'alumine de manière à obtenir, après un séchage à une température égale à 100°C pendant une nuit et une calcination sous air sec menée à une température égale à 450°C pendant 4 heures, le support S1 qui contient en poids 10 % de zéolithe EU-1 et 90 % d'alumine.
Ce support S1 est soumis à un échange anionique avec de l'acide hexachloroplatinique en présence d'acide chlorhydrique en tant qu'agent compétiteur, de manière à déposer 0,5 % poids de platine par rapport au poids du catalyseur. Le solide humide est ensuite séché à 120°C pendant 12 heures et calciné sous un débit d'air sec à la température de 500°C pendant une heure.
Le catalyseur A ainsi obtenu contient, en poids, 10 % de zéolithe EU-1, 89,5 % d'alumine et 0,5 % de platine.

### Exemple 5 (invention) : Préparation du catalyseur B comprenant une zéolithe EU-1 modifiée

La zéolithe EU-1(2) obtenue à l'exemple 2 est ensuite mise en forme par extrusion avec un gel d'alumine de manière à obtenir, après un séchage à une température égale à 100°C pendant une nuit et une calcination sous air sec menée à une température égale à 450°C pendant 4 heures, le support S2 qui contient en poids 10 % de zéolithe EU-1 modifiée et 90 % d'alumine.
Ce support S2 est soumis à un échange anionique avec de l'acide hexachloroplatinique en présence d'acide chlorhydrique en tant qu'agent compétiteur, de manière à déposer 0,5 % poids de platine par rapport au catalyseur. Le solide humide est ensuite séché à 120°C pendant 12 heures et calciné sous un débit d'air sec à la température de 500°C pendant une heure.
Le catalyseur B ainsi obtenu contient, en poids, 10 % de zéolithe EU-1 modifiée, 89,5 % d'alumine et 0,5 % de platine.

### Exemple 6 (invention) : Préparation du catalyseur C comprenant une zéolithe EU-1 modifiée

La zéolithe EU-1(3) obtenue à l'exemple 3 est ensuite mise en forme par extrusion avec un gel d'alumine de manière à obtenir, après un séchage à une température égale à 100°C pendant une nuit et une calcination sous air sec menée à une température égale à 450°C pendant 4 heures, le support S3 qui contient en poids 10 % de zéolithe EU-1 modifiée et 90 % d'alumine.
Ce support S3 est soumis à un échange anionique avec de l'acide hexachloroplatinique en présence d'acide chlorhydrique en tant qu'agent compétiteur, de manière à déposer 0,5 % poids de platine par rapport au poids de catalyseur. Le solide humide est ensuite séché à 120°C pendant 12 heures et calciné sous un débit d'air sec à la température de 500°C pendant une heure.
Le catalyseur C ainsi obtenu contient, en poids, 10 % de zéolithe EU-1 modifiée, 89,5 % d'alumine et 0,5 % de platine.

### Exemple 7: Évaluation des propriétés catalytiques du catalyseur A, B et C en isomérisation de l'ethylbenzène.

La charge à isomériser, mise en contact avec le catalyseur A, le catalyseur B puis le catalyseur C, est constituée uniquement d'éthylbenzène.
Les conditions opératoires de l'isomérisation sont les suivantes :
- température : 410°C;
- pression totale : 10 bar (1 bar = 0,1 MPa) ;
- pression partielle d'hydrogène : 8 bar.
- charge : ethylbenzène
- vitesse spatiale d'alimentation, exprimée en kilogramme de charge introduite par kilogramme de catalyseur et par heure, égale à 8,7 h⁻¹.

On évalue successivement les propriétés catalytiques des catalyseurs A, B et C pour l'isomérisation de l'éthylbenzène. Chacun des catalyseurs est réduit sous hydrogène pendant 4 heures à 480°C avant injection de la charge.
Les catalyseurs ont été évalués en termes de conversion d'éthylbenzène et de sélectivité en xylènes.

La sélectivité en xylènes est calculée au moyen du rendement en xylènes produits. Le rendement en xylènes est déterminé à partir du % massique des xylènes produits, obtenu par analyse de chaque effluent.

La conversion de l'éthylbenzène est le pourcentage d'éthylbenzène consommé.

**Tableau 1 : Conversion de l'éthylbenzène et sélectivité en xylènes sur les catalyseurs A, B et C après 4000 min de réaction**

| | catalyseur A | catalyseur B | catalyseur C |
|---|---|---|---|
| conversion éthylbenzène(%) | 28,6 | 41,8 | 42,3 |
| sélectivité en xylènes (%) | 68,1 | 68,1 | 68,1 |
| Rendement en xylènes (%) | 19,5 | 28,5 | 28,8 |

Les résultats présentés dans le tableau 1 montrent que les catalyseurs B et C comprenant chacun une zéolithe EU-1 modifiée, dans lesquelles le nombre d'atomes d'aluminium hexacoordinés extra-réseau représentent plus de 20% poids du nombre total des atomes d'aluminium présents dans chacune desdites zéolithes modifiées, conduit à de bien meilleures performances catalytiques en terme de conversion d'éthylbenzène que celle obtenue au moyen du catalyseur A comprenant une zéolithe EU-1 non modifiée. Les catalyseurs B et C selon l'invention sont donc sensiblement plus actifs que le catalyseur A de l'état de la technique.
Par ailleurs, les catalyseurs B et C selon l'invention conduisent à une sélectivité en xylènes identique à celle obtenue avec le catalyseur A, en conséquence les catalyseurs B et C selon l'invention conduisent à un rendement en xylènes bien supérieur au rendement en xylènes obtenu avec le catalyseur comparatif A, le rendement en xylènes étant le produit de la conversion de l'éthylbenzène par la sélectivité en xylènes.

## Revendications

1. Catalyseur comprenant au moins une zéolithe EU-1 modifiée contenant des atomes de silicium et des atomes d'aluminium, au moins une matrice et au moins un métal du groupe VIII de la classification périodique des éléments, ledit catalyseur étant **caractérisé en ce que** le nombre d'atomes d'aluminium hexacoordinés présents dans ladite zéolithe modifiée représente plus de 20% poids du nombre total d'atomes d'aluminium présents dans ladite zéolithe EU-1 modifiée.

2. Catalyseur selon la revendication 1 tel que ladite zéolithe EU-1 modifiée présente un nombre d'atomes d'aluminium hexacoordinés représentant plus de 22% poids du nombre total d'atomes d'aluminium présents dans ladite zéolithe modifiée.

3. Catalyseur selon la revendication 1 tel que ladite zéolithe EU-1 modifiée présente un nombre d'atomes d'aluminium hexacoordinés représentant plus de 25% poids du nombre total d'atomes d'aluminium présents dans ladite zéolithe modifiée.

4. Catalyseur selon la revendication 1 tel que ladite zéolithe EU-1 modifiée présente un nombre d'atomes d'aluminium hexacoordinés représentant plus de 30% poids du nombre total d'atomes d'aluminium présents dans ladite zéolithe modifiée.

5. Catalyseur selon l'une des revendications 1 à 4 tel que ladite zéolithe EU-1 modifiée présente un rapport atomique Si/Al global compris entre 10 et 35.

6. Catalyseur selon l'une des revendications 1 à 5 tel que ledit métal du groupe VIII est le platine.

7. Catalyseur selon l'une des revendications 1 à 6 tel qu'il comprend au moins un métal additionnel choisi dans le groupe formé par les métaux des groupes IIIA, IVA et VIIB.

8. Catalyseur selon l'une des revendications 1 à 7 tel qu'il comprend du soufre.

9. Catalyseur selon l'une des revendications 1 à 8 tel que ladite matrice est une alumine.

10. Catalyseur selon l'une des revendications 1 à 9 tel qu'il se présente sous forme d'extrudés.

11. Procédé de préparation d'un catalyseur selon l'une des revendications 1 à 10 comprenant au moins les étapes suivantes :
a1) la synthèse d'au moins une zéolithe EU-1 ayant un rapport atomique Si/Al global compris entre 5 et 100,
b1) le traitement thermique en présence de vapeur d'eau de la zéolithe issue de ladite étape a1) de manière à obtenir une zéolithe EU-1 modifiée,
c1) la mise en forme de ladite zéolithe EU-1 modifiée avec une matrice pour former un support zéolithique modifié,
d1) le dépôt d'au moins un métal du groupe VIII de la classification périodique des éléments, l'ordre de réalisation desdites étapes c1) et d1) étant indifférent à la suite de ladite étape b1).

12. Procédé de préparation selon la revendication 11 tel que ledit traitement thermique en présence de vapeur d'eau est réalisé à une température comprise entre 200 et 470°C, pendant une durée comprise entre 0,5 heure et 24 heures, le pourcentage volumique de vapeur d'eau étant compris entre 5 et 100%.

13. Procédé de préparation selon la revendication 11 ou la revendication 12 tel que ladite étape c1) précède ladite étape d1).

14. Procédé de préparation d'un catalyseur selon l'une des revendications 1 à 10 comprenant au moins les étapes suivantes :
a2) la synthèse d'au moins une zéolithe EU-1 ayant un rapport atomique Si/Al global compris entre 5 et 100,
b2) la mise en forme de la zéolithe EU-1 issue de ladite étape a2) avec une matrice pour former un support zéolithique,
c2) le traitement thermique en présence de vapeur d'eau du support zéolithique formé à l'étape b2),
d2) le dépôt d'au moins un métal du groupe VIII de la classification périodique des éléments sur le support zéolithique modifié selon l'étape c2).

15. Procédé de préparation d'un catalyseur selon l'une des revendications 1 à 10 comprenant au moins les étapes suivantes :
a3) la synthèse d'au moins une zéolithe EU-1 ayant un rapport atomique Si/Al global compris entre 5 et 100,
b3) la mise en forme de la zéolithe EU-1 issue de ladite étape a3) avec une matrice pour former un support zéolithique,
c3) le dépôt d'au moins un métal du groupe VIII de la classification périodique des éléments sur le support zéolithique issu de ladite étape b3),
d3) le traitement thermique en présence de vapeur d'eau du support zéolithique imprégné d'au moins dudit métal du groupe VIII issu de ladite étape c3).

16. Procédé d'isomérisation d'une coupe contenant au moins un composé aromatique à huit atomes de carbone par molécule, ledit procédé comprenant la mise en contact de ladite coupe aromatique avec au moins un catalyseur comprenant une zéolithe modifiée selon l'une des revendications 1 à 10 ou préparé selon l'une des revendications 11 à 15 et présent dans un réacteur catalytique.

17. Procédé d'isomérisation selon la revendication 16 tel qu'il est mis en oeuvre dans les conditions opératoires suivantes : une température comprise entre 300 et 500°C, une pression partielle d'hydrogène comprise entre 0,3 et 1,5 MPa, une pression totale comprise entre 0,45 et 1,9 MPa et une vitesse spatiale d'alimentation, exprimée en kilogramme de charge introduite par kilogramme de catalyseur et par heure, comprise entre 0,25 et 30 h⁻¹.

18. Procédé d'isomérisation selon la revendication 16 ou la revendication 17 tel que la zéolithe EU-1 présente dans ledit catalyseur a été modifiée par un traitement thermique en présence de vapeur d'eau, ledit traitement étant réalisé *in-situ* et préalablement à la mise en contact dudit catalyseur avec ladite coupe aromatique.

## Claims

1. A catalyst comprising at least one modified EU-1 zeolite containing silicon atoms and aluminium atoms, at least one matrix and at least one metal from group VIII of the periodic table of the elements, said catalyst being **characterized in that** the number of hexacoordinated aluminium atoms present in said modified zeolite represents more than 20% by weight of the total number of aluminium atoms present in said modified EU-1 zeolite.

2. A catalyst according to claim 1, in which said modified EU-1 zeolite has a number of hexacoordinated aluminium atoms representing more than 22% by weight of the total number of aluminium atoms present in said modified zeolite.

3. A catalyst according to claim 1, in which said modified EU-1 zeolite has a number of hexacoordinated aluminium atoms representing more than 25% by weight of the total number of aluminium atoms present in said modified zeolite.

4. A catalyst according to claim 1, in which said modified EU-1 zeolite has a number of hexacoordinated aluminium atoms representing more than 30% by weight of the total number of aluminium atoms present in said modified zeolite.

5. A catalyst according to one of claims 1 to 4, in which said modified EU-1 zeolite has an overall Si/A1 atomic ratio in the range 10 to 35.

6. A catalyst according to one of claims 1 to 5, in which said group VIII metal is platinum.

7. A catalyst according to one of claims 1 to 6, comprising at least one additional metal selected from the group formed by metals from groups IIIA, IVA and VIIB.

8. A catalyst according to one of claims 1 to 7, comprising sulphur.

9. A catalyst according to one of claims 1 to 8, in which said matrix is an alumina.

10. A catalyst according to one of claims 1 to 9, in which it is in the form of extrudates.

11. A process for preparing a catalyst according to one of claims 1 to 10, comprising at least the following steps:
a1) synthesizing at least one EU-1 zeolite having an overall Si/A1 atomic ratio in the range 5 to 100;
b1) heat treating the zeolite from said step a1) in the presence of steam to obtain a modified EU-1 zeolite;
c1) forming said modified EU-1 zeolite with a matrix to form a modified zeolitic support;
d1) depositing at least one metal from group VIII of the periodic table of the elements, the order of carrying out said steps c1) and d1) being inconsequential following upon said step b1).

12. A preparation process according to claim 11, in which said heat treatment in the presence of steam is carried out at a temperature in the range 200°C to 470°C, for a period in the range 0.5 hours to 24 hours, the percentage by volume of steam being in the range 5% to 100%.

13. A preparation process according to claim 11 or claim 12, in which said step c1) precedes said step d1).

14. A process for preparing a catalyst according to one of claims 1 to 10, comprising at least the following steps:
a2) synthesizing at least one EU-1 zeolite having an overall Si/A1 atomic ratio in the range 5 to 100;
b2) forming the EU-1 zeolite from said step a2) with a matrix to form a zeolitic support;
c2) heat treating the zeolitic support formed in step b2) in the presence of steam;
d2) depositing at least one metal from group VIII of the periodic table of the elements on the modified zeolitic support of step c2).

15. A process for preparing a catalyst according to one of claims 1 to 10, comprising at least the following steps:
a3) synthesizing at least one EU-1 zeolite having an overall Si/A1 atomic ratio in the range 5 to 100;
b3) forming the EU-1 zeolite from said step a3) with a matrix to form a zeolitic support;
c3) depositing at least one metal from group VIII of the periodic table of the elements on the zeolitic support from said step b3);
d3) heat treating the zeolitic support impregnated with at least said group VIII metal from said step c3) in the presence of steam.

16. A process for isomerizing a cut containing at least one aromatic compound containing eight carbon atoms per molecule, said process comprising bringing said aromatic cut into contact with at least one catalyst comprising a modified zeolite according to one of claims 1 to 10 or prepared in accordance with one of claims 11 to 15 and present in a catalytic reactor.

17. An isomerization process according to claim 16, carried out under the following operating conditions: a temperature in the range 300°C to 500°C, a partial pressure of hydrogen in the range 0.3 to 1.5 MPa, a total pressure in the range 0.45 to 1.9 MPa and a space velocity, expressed in kilograms of feed introduced per kilogram of catalyst per hour, in the range 0.25 to 30 h⁻¹.

18. An isomerization process according to claim 16 or claim 17, in which the EU-1 zeolite present in said catalyst has been modified by a heat treatment in the presence of steam, said treatment being carried out in situ and prior to bringing said catalyst into contact with said aromatic cut.

## Patentansprüche

1. Katalysator, der mindestens einen modifizierten EU-1-Zeolithen, der Siliciumatome und Aluminiumatome enthält, mindestens eine Matrix und mindestens ein Metall der Gruppe VIII des Periodensystems der Elemente umfasst, wobei der Katalysator **dadurch gekennzeichnet ist, dass** die Anzahl der sechsfach koordinativ gebundenen Aluminiumatome, die in dem modifizierten Zeolithen vorhanden sind, mehr als 20 Gew.-% der Gesamtanzahl an Aluminiumatomen darstellt, die in dem modifizierten EU-1-Zeolithen vorhanden sind.

2. Katalysator nach Anspruch 1, wobei der modifizierte EU-1-Zeolith eine Anzahl der sechsfach koordinativ gebundenen Aluminiumatomen aufweist, die mehr als 22 Gew.-% der Gesamtanzahl an Aluminiumatomen darstellt, die in dem modifizierten Zeolithen vorhanden sind.

3. Katalysator nach Anspruch 1, wobei der modifizierte EU-1-Zeolith eine Anzahl der sechsfach koordinativ gebundenen Aluminiumatomen aufweist, die mehr als 25 Gew.-% der Gesamtanzahl an Aluminiumatomen darstellt, die in dem modifizierten Zeolithen vorhanden sind.

4. Katalysator nach Anspruch 1, wobei der modifizierte EU-1-Zeolith eine Anzahl der sechsfach koordinativ gebundenen Aluminiumatomen aufweist, die mehr als 30 Gew.-% der Gesamtanzahl an Aluminiumatomen darstellt, die in dem modifizierten Zeolithen vorhanden sind.

5. Katalysator nach einem der Ansprüche 1 bis 4, wobei der modifizierte EU-1-Zeolith ein Si/Al-Gesamtatomverhältnis im Bereich zwischen 10 und 35 aufweist.

6. Katalysator nach einem der Ansprüche 1 bis 5, wobei das Metall der Gruppe VIII Platin ist.

7. Katalysator nach einem der Ansprüche 1 bis 6, wobei er mindestens ein zusätzliches Metall umfasst, das ausgewählt ist aus der Gruppe gebildet aus den Metallen der Gruppen IIIA, IVA und VIIB.

8. Katalysator nach einem der Ansprüche 1 bis 7, wobei er Schwefel umfasst.

9. Katalysator nach einem der Ansprüche 1 bis 8, wobei die Matrix ein Aluminiumoxid ist.

10. Katalysator nach einem der Ansprüche 1 bis 9, der die Form von Extrudaten aufweist.

11. Verfahren zur Herstellung eines Katalysators nach einem der Ansprüche 1 bis 10, das mindestens die folgenden Schritte umfasst:
a1) Synthese mindestens eines EU-1-Zeolithen, der ein Si/Al-Gesamtatomverhältnis im Bereich zwischen 5 und 100 hat,
b1) thermische Behandlung des Zeolithen, der aus Schritt a1) stammt, in Gegenwart von Wasserdampf, um einen modifizierten EU-1-Zeolithen zu erhalten,
c1) Formen des modifizierten EU-1-Zeolithen mit einer Matrix, um einen modifizierten zeolithischen Träger zu bilden,
d1) Abscheiden mindestens eines Metalls der Gruppe VIII des Periodensystems der Elemente, wobei die Ausführungsreihenfolge der Schritte c1) und d1) nach dem Schritt b1) unwesentlich ist.

12. Verfahren zur Herstellung nach Anspruch 11, wobei die thermische Behandlung in Gegenwart von Wasserdampf bei einer Temperatur im Bereich zwischen 200 und 470 °C, während einer Dauer im Bereich zwischen 0,5 Stunden und 24 Stunden ausgeführt wird, wobei der Volumenprozentsatz des Wasserdampfs im Bereich zwischen 5 und 100 % liegt.

13. Verfahren zur Herstellung nach Anspruch 11 oder Anspruch 12, wobei der Schritt c1) dem Schritt d1) vorausgeht.

14. Verfahren zur Herstellung eines Katalysators nach einem der Ansprüche 1 bis 10, das mindestens die folgenden Schritte umfasst:
a2) Synthese mindestens eines EU-1-Zeolithen, der ein Si/Al-Gesamtatomverhältnis im Bereich zwischen 5 und 100 hat,
b2) Formen des EU-1-Zeolithen, der aus dem Schritt a2) stammt, mit einer Matrix, um einen zeolithischen Träger zu bilden,
c2) thermische Behandlung des zeolithischen Trägers, der in Schritt b2) gebildet wurde, in Gegenwart von Wasserdampf,
d2) Abscheiden mindestens eines Metalls der Gruppe VIII des Periodensystems der Elemente auf dem zeolithischen Träger, der gemäß Schritt c2) modifiziert wurde.

15. Verfahren zur Herstellung eines Katalysators nach einem der Ansprüche 1 bis 10, das mindestens die folgenden Schritte umfasst:
a3) Synthese mindestens eines EU-1-Zeolithen, der ein Si/Al-Gesamtatomverhältnis im Bereich zwischen 5 und 100 hat,
b3) Formen des EU-1-Zeolithen, der aus dem Schritt a3) stammt, mit einer Matrix, um einen zeolithischen Träger zu bilden,
c3) Abscheiden mindestens eines Metalls der Gruppe VIII des Periodensystems der Elemente auf dem zeolithischen Träger, der aus dem Schritt b3) stammt.
d3) thermische Behandlung des zeolithischen Trägers, der mit mindestens dem Metall der Gruppe VIII imprägniert ist, der aus dem Schritt c3) stammt, in Gegenwart von Wasserdampf.

16. Verfahren zur Isomerisation eines Schnitts, der mindestens eine aromatische Verbindung mit acht Kohlenstoffatomen pro Molekül hat, wobei das Verfahren das Inkontaktbringen des aromatischen Schnitts mit mindestens einem Katalysator umfasst, der einen modifizierten Zeolithen nach einem der Ansprüche 1 bis 10 umfasst oder der nach einem der Ansprüche 11 bis 15 hergestellt wurde und in einem katalytischen Reaktor vorhanden ist.

17. Verfahren zur Isomerisation nach Anspruch 16, wobei es unter den folgenden Betriebsbedingungen durchgeführt wird: einer Temperatur im Bereich zwischen 300 und 500 °C, einem Wasserstoffpartialdruck im Bereich zwischen 0,3 und 1,5 MPa, einem Gesamtdruck im Bereich zwischen 0,45 und 1,9 MPa und einer Versorgungsraumgeschwindigkeit, ausgedrückt in Kilogramm eingeführter Beschickung pro Kilogramm Katalysator und pro Stunde, im Bereich zwischen 0,25 und 30 h⁻¹.

18. Verfahren zur Isomerisation nach Anspruch 16 oder Anspruch 17, wobei der EU-1-Zeolith, der in dem Katalysator vorhanden ist, durch eine thermische Behandlung in Gegenwart von Wasserdampf modifiziert wurde, wobei die Behandlung *in situ* und vor dem Inkontaktbringen des Katalysators mit dem aromatischen Schnitt ausgeführt wird.
